# EUROPÄISCHE PATENTANMELDUNG

(11) **EP 0 532 965 A2**
(43) Veröffentlichungstag der Anmeldung: **24.03.1993**
(21) Anmeldenummer: 92114811.0
(22) Anmeldetag: 29.08.1992
(51) Int. Cl.: C07C 45/71, C07C 49/84, C07C 315/04, C07C 317/22

(54) **Verfahren zur Herstellung von aromatischen Carbonyl- oder Sulfonylverbindugen mit Aryletherstruktur**

(30) Priorität: 19.09.1991 DE 4131144
(71) Anmelder: BASF Aktiengesellschaft, D-67063 Ludwigshafen (DE)
(72) Erfinder: Hahn, Erwin, Dr., W-6900 Heidelberg (DE); Eilingsfeld, Heinrich Johann, Dr., W-6710 Frankenthal (DE); Reichelt, Helmut, Dr., W-6730 Neustadt (DE); Aumueller, Alexander, Dr., W-6730 Neustadt (DE); Hupfeld, Bernd, Dr., W-6720 Speyer (DE)

(57) **Zusammenfassung**

Verfahren zur Herstellung von aromatischen Carbonyl- oder Sulfonylverbindungen mit Diaryletherstruktur durch Umsetzung von Phenolen mit halogenierten Carbonyl- oder Sulfonylaromaten in einem dipolar aprotischen Lösungsmittel, wobei man in Gegenwart von katalytischen Mengen von Alkalinitrit oder einer aromatischen Nitro-, Nitroso-, Azo-, Azoxy- oder Hydrazoverbindung arbeitet.

## Beschreibung

Die vorliegende Erfindung betrifft ein neues Verfahren zur Herstellung von aromatischen Carbonyl- oder Sulfonylverbindungen mit Aryletherstruktur durch Umsetzung von Phenolen mit halogenierten Carbonyl- oder Sulfonylaromaten in einem dipolar aprotischen Lösungsmittel.

Aromatische Carbonyl oder Sulfonylverbindungen mit Aryletherstruktur (z.B. 4,4'-Diphenoxybenzophenon) werden für die Herstellung von hochtemperaturbeständigen Polymeren, z.B. von Polyetherketonen, benötigt. Zu diesem Zweck werden sie in hochreiner Form verwendet.

Aus der EP-A 262 919 ist beispielsweise die Herstellung von solchen Verbindungen durch Umsetzung von Diphenylether mit Halogenbenzoylhalogeniden bekannt. Nachteilig an diesem Verfahren ist einerseits die Bildung von Nebenprodukten (Oligomere) sowie die Tatsache, daß zur Herstellung der Halogenbenzoylhalogeniden Phosgen verwendet werden muß.

Weiterhin beschreibt die EP-A 282 096 eine Herstellweise, bei der Hydroxyarylverbindungen mit Halogenaromaten umgesetzt werden. Nachteilig bei dieser Verfahrensweise ist die geringe Reaktivität von Chloraromaten. Dies hat zur Folge, daß Fluoraromaten, die sehr viel teurer sind als Chloraromaten, zur Anwendung kommen müssen.

Aufgabe der vorliegenden Erfindung war es nun, ein neues Verfahren zur Herstellung von aromatischen Carbonyl- oder Sulfonylverbindungen mit Aryletherstruktur bereitzustellen, das in einfacher Weise durchgeführt werden kann. Im neuen Verfahren sollten Hydroxyarylverbindungen mit den reaktionsträgen Chlor- oder Bromaromaten umgesetzt werden. Dabei sollten die Zielprodukte in hoher Ausbeute und Reinheit anfallen.

Es wurde nun gefunden, daß die Herstellung von Arylethern der Formel I 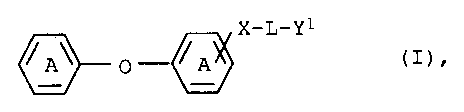
in der
der Ring A gegebenenfalls substituiert ist und benzoanelliert sein kann,
- X: Carbonyl oder Sulfonyl,
- L: eine chemische Bindung oder ein Brückenglied und
- Y¹: a) für den Fall, daß L für ein Brückenglied steht, Halogen oder den Rest der Formel 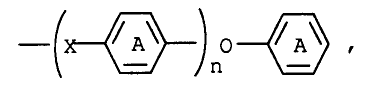 worin n die Bedeutung von 0 oder 1 und X und der Ring A jeweils die obengenannte Bedeutung besitzen,
b) für den Fall, daß L für eine chemische Bindung und X für Carbonyl stehen, Wasserstoff, C₁-C₄-Alkyl, C₁-C₄-Alkoxy oder den Rest der Formel 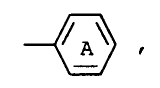 worin der Ring A die obengenannte Bedeutung besitzt, oder
c) für den Fall, daß L für eine chemische Bindung und X für Sulfonyl stehen, den Rest der Formel 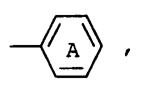 worin der Ring A die obengenannte Bedeutung besitzt,

bedeuten, durch Umsetzung von Hydroxyaromaten der Formel II 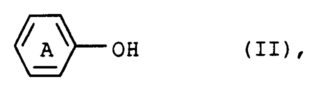
in der der Ring A die obengenannte Bedeutung besitzt, mit Halogenverbindungen der Formel III 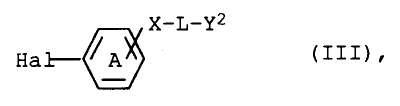
in der
Hal Chlor oder Brom und
- Y²: einen Rest der Formel 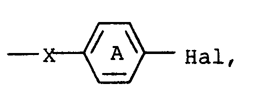
worin X, Hal und der Ring A jeweils die obengenannte Bedeutung besitzen, oder den obengenannten Rest Y¹ bedeuten und X, Hal und der Ring jeweils die obengenannte Bedeutung besitzen, in einem dipolar aprotischen Lösungsmittel in Gegenwart einer Base vorteilhaft gelingt, wenn man die Umsetzung bei einer Temperatur von 60 bis 240°C zusätzlich in Gegenwart einer katalytischen Menge einer Verbindung vornimmt, die ausgewählt ist aus der Klasse, bestehend aus Alkalinitrit und Verbindungen der Formel IV 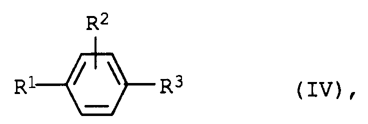
in der
- R¹ und R²: gleich oder verschieden sind und unabhängig voneinander jeweils Wasserstoff, C₁-C₁₂-Alkyl, das gegebenenfalls durch Phenyl substituiert ist, C₁-C₁₂-Alkoxy, Halogen oder Nitro und
- R³: Nitro, Nitroso oder einen Rest der Formel 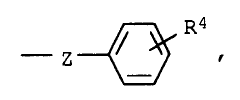 worin R⁴ für Wasserstoff, C₁-C₄-Alkyl oder Halogen und Z für einen Rest der Formel -N=N, 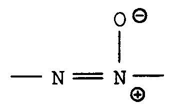
oder -NH-NH- stehen, bedeuten, mit der Maßgabe, daß wenn R³ für Nitro steht, mindestens einer der beiden Reste R¹ und R² die Bedeutung von Halogen besitzt.

Alle in Formel I, II oder III auftretenden Alkylgruppen können sowohl geradkettig als auch verzweigt sein.

Wenn in Formel I, II oder III substituierte Phenyl- oder Naphthylgruppen auftreten, so können als Substituenten z.B. C₁-C₄-Alkyl, Hydroxy, C₁-C₄-Alkoxy, Phenyl oder durch C₁-C₄-Alkyl oder C₁-C₄-Alkoxy substituiertes Phenyl in Betracht kommen. Die Ringe können dabei in der Regel ein- bis dreifach substituiert sein.

Wenn Y¹ Halogen bedeutet, so kommt dafür z.B. Fluor oder insbesondere Chlor oder Brom in Betracht.

Wenn L für ein Brückenglied steht, so kann dafür z.B. Phenylen, Biphenylen, Naphthylen oder ein Rest der Formel 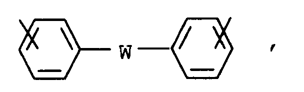
in der W Sauerstoff, Schwefel, Carbonyl oder Sulfonyl bedeutet, in Betracht kommen.

Reste R¹, R² und R⁴ sind z.B. Methyl, Ethyl, Propyl, Isopropyl, Butyl, Isobutyl, sec-Butyl, Fluor, Chlor oder Brom.

Reste R¹ und R² sind weiterhin z.B. Pentyl, Isopentyl, Neopentyl, tert-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, (Die Bezeichnungen Isooctyl, Isononyl und Isodecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436.), Benzyl, 1- oder 2-Phenylethyl, Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, 2-Methylpentyloxy, Heptyloxy, 1-Ethylpentyloxy, Octyloxy, 2-Ethylhexyloxy, Isooctyloxy, Nonyloxy, Decyloxy, Isodecyloxy, Undecyloxy oder Dodecyloxy.

Geeignete dipolar aprotische Lösungsmittel, die im erfindungsgemäßen Verfahren zur Anwendung kommen können, sind z.B. Aceton, Acetonitril, Nitromethan, N,N-Dimethylformamid, N, N-Dimethylacetamid, N-Methylpyrrolidinon, Tetramethylharnstoff, Hexamethylphosphorsäuretriamid, Dimethylsulfoxid, Tetrahydrothiophen-1,1-dioxid (Sulfolan), Diether des Ethylenglykols, wie Ethylenglykoldimethyl- oder -diethylether, oder Ethylencarbonat.

Geeignete Basen, die im erfindungsgemäßen Verfahren zur Anwendung gelangen können, sind z.B. Alkalicarbonate, wie Natrium- oder Kaliumcarbonat oder deren Gemische.

Wenn das erfindungsgemäße Verfahren in Gegenwart eines Alkalinitrits durchgeführt wird, so kommt dafür z.B. Natrium- oder Kaliumnitrit in Betracht.

Hervorzuheben ist eine Verfahrensweise zur Herstellung von Arylethern der Formel I, in der Ring A unsubstituiert, durch Phenyl substituiert oder benzoanelliert ist.

Hervorzuheben ist weiterhin eine Verfahrensweise zur Herstellung von Arylethern der Formel I, in der L ein Brückenglied bedeutet.

Hervorzuheben ist weiterhin eine Verfahrensweise zur Herstellung von Arylethern der Formel I, in der man Hydroxyaromaten der Formel II mit Halogenverbindungen der Formel III, in der Hal Chlor bedeutet, umsetzt.

Von besonderem Interesse ist eine Verfahrensweise zur Herstellung von Arylethern der Formel I, in der L Phenylen, insbesondere 1,4-Phenylen, oder Biphenylen, insbesondere 4-4'-Biphenylen bedeutet.

Bevorzugt ist eine Verfahrensweise zur Herstellung von Arylethern der Formel Ia 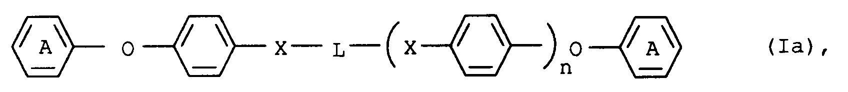
in der
der Ring A unsubstituiert, durch Phenyl substituiert oder benzoanelliert ist,
- X: Carbonyl oder Sulfonyl,
- L: Phenylen oder Biphenylen und
n 0 oder 1 bedeuten, durch Umsetzung von Hydroxyaromaten der Formel II 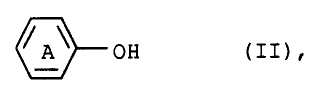
in der der Ring A die zuletzt genannte Bedeutung besitzt, mit Halogenverbindungen der Formel IIIa 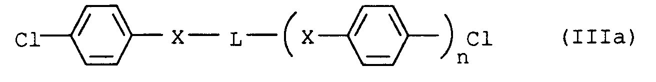
in der X, L und n jeweils die obengenannte Bedeutung besitzen.

Besonders bevorzugt ist eine Verfahrensweise zur Herstellung von Arylethern der Formel Ib 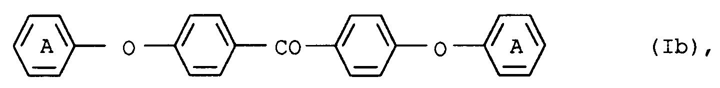
in der der Ring A unsubstituiert, durch Phenyl substituiert oder benzoanelliert ist, durch Umsetzung von Hydroxyaromaten der Formel II 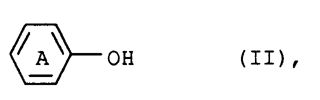
in der der Ring A die zuletzt genannte Bedeutung besitzt, mit 4,4'-Dichlorbenzophenon.

Besonders hervorzuheben ist weiterhin eine Verfahrensweise zur Herstellung von Arylethern der Formel Ic 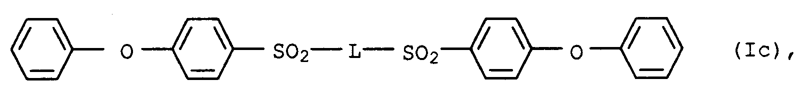
in der L Phenylen oder Naphthylen bedeutet, durch Umsetzung von Phenol mit Halogenverbindungen der Formel IIIb 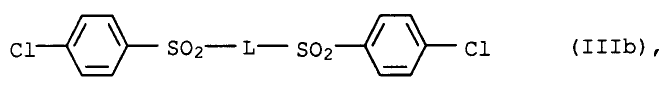
in der L die zuletzt genannte Bedeutung besitzt.

Wichtig ist eine Verfahrensweise, in der man die Umsetzung in Gegenwart einer Verbindung der Formel IV durchführt.

Von besonderer Bedeutung ist eine Verfahrensweise, in der man die Umsetzung in Gegenwart einer Verbindung der Formel IV durchführt, in der
- R¹: Wasserstoff,
- R²: Wasserstoff oder Halogen und
- R³: Nitro oder einen Rest der Formel 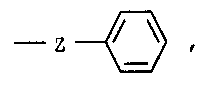 worin Z die obengenannte Bedeutung besitzt, bedeuten.

Als Verbindungen, die der Formel IV gehorchen, sind insbesondere 1-Chlor-2-nitrobenzol, 1-Chlor-3-nitrobenzol, Azobenzol, Azoxybenzol oder Hydrazobenzol zu nennen.

Besonders geeignete dipolare aprotische Lösungsmittel im erfindungsgemäßen Verfahren sind N,N-Dimethylformamid oder insbesondere N-Methylpyrrolidinon.

Als Base ist Kaliumcarbonat im erfindungsgemäßen Verfahren besonders geeignet.

Das neue Verfahren wird bei einer Temperatur von 60 bis 240°C, vorzugsweise 80 bis 200°C und üblicherweise unter Normaldruck durchgeführt.

Das Molverhältnis Hydroxyaromat II:Halogenverbindung III (als Monohalogenverbindung) beträgt in der Regel 2:1, vorzugsweise 1,05:1. Wenn man als Halogenverbindung III eine Dihalogenverbindung verwendet, beträgt das obengenannte Molverhältnis in der Regel 4:1 vorzugsweise 2,1:1.

Das Molverhältnis Base:Hydroxyaromat II beträgt in der Regel 1,5:1 bis 1:1, vorzugsweise ca. 1:1.

Die Verbindung IV kommt in katalytischen Mengen zur Anwendung, darunter können im erfindungsgemäßen Sinn 0,1 bis 20 Mol-%, vorzugsweise 1 bis 10 Mol-%, jeweils bezogen auf die Halogenverbindung III, verstanden werden. Eine Erhöhung dieser Menge ist möglich, bringt jedoch keine Vorteile.

Die Menge an dipolare aprotischem Lösungsmittel beträgt im neuen Verfahren in der Regel 200 bis 1000 Gew.-%, bezogen auf das Gewicht von Hydroxyaromat II.

Das erfindungsgemäße Verfahren kann sowohl in kontinuierlicher als auch diskontinuierlicher Arbeitsweise vorgenommen werden. Es wird zweckmäßig so durchgeführt, daß man Lösungsmittel, Hydroxyaromat II, Halogenverbindung III, Base und Alkalinitrit oder Verbindung IV vorlegt und unter Rühren und gegebenenfalls unter einer Schutzgasatmosphäre (z.B. Stickstoff oder Helium) auf die obengenannte Temperatur erhitzt. Es erfolgt dann eine Nachrührphase bei dieser Temperatur, die im allgemeinen 4 bis 10 Stunden in Anspruch nimmt.

Zur Aufarbeitung wird das Reaktionsgemisch auf eine Temperatur von ca. 10 bis 70°C abgekühlt und dann in Wasser eingetragen. Dabei fällt der Arylether I als Niederschlag aus und kann z.B. durch Filtration abgetrennt, mit Wasser gewaschen und getrocknet werden.

Ein Vorteil des neuen Verfahrens ist darin zu sehen, daß es auf technisch einfache Weise durchgeführt werden kann, wobei reaktionsträge Chlor- oder Bromverbindungen zur Umsetzung gelangen können. Die Arylether der Formel I fallen dabei in guter Ausbeute und hoher Reinheit an. Dies ist von besonderer Bedeutung, da wie oben bereits ausgeführt, die Zielverbindungen als Zwischenprodukte für die Herstellung von hochtemperaturbeständigen Polymeren dienen und zu diesem Zweck in hochreiner Form vorliegen müssen.

Die folgenden Beispiele sollen die Erfindung näher erläutern.

### Beispiel 1

### Herstellung von 4,4'-Diphenoxybenzophenon (Vergleich)

In einem 500 ml Kolben mit Destillationsaufsatz, Rührer und Rückflußkühler wurden unter Stickstoffatmosphäre bei Raumtemperatur 110 ml N-Methylpyrrolidinon (NMP), 24,2 g (0,257 mol) Phenol, 31,38 g (0,125 mol) 4,4'-Dichlorbenzophenon und 35,5 g (0,257 mol) Kaliumcarbonat vorgelegt und die Reaktionsmischung anschließend auf 190 bis 195°C erhitzt. Bei 185 bis 190°C wurden ca. 5 bis 6 ml Wasser/NMP-Gemisch abdestilliert. Der Reaktionsverlauf wurde mittels Dünnschichtchromatographie verfolgt. Nach 10 Stunden Reaktionszeit bei 190°C wurde kein weiterer Umsatz beobachtet (im DC war kein Edukt mehr nachweisbar). Die Reaktionslösung wurde auf ca. 80°C abgekühlt und in 350 ml Wasser unter gutem Rühren eingetragen. Vom ausgefallenen Produkt wurde abfiltriert, der Filterrückstand mit 1000 ml Wasser gewaschen und unter vermindertem Druck getrocknet. Eine HPLC-Analyse einer Probe zeigte 76,5 % 4,4'-Diphenoxybenzophenon, 19,2 % 4-Chlor-4'-phenoxybenzophenon und 4,3 % nicht identifizierter Verunreinigungen.

### Beispiele 2 bis 7 (erfindungsgemäß)

Man verfuhr analog Beispiel 1, führte jedoch die Umsetzung in Gegenwart der in der folgenden Tabelle 1 aufgeführten Katalysatoren durch.

**Tabelle 1**

| Beispiel Nr. | Katalysator | Reaktionszeit [h] | Ausbeute [%] | Reinheit (HPLC) [%] |
|---|---|---|---|---|
| 2 | 1-Chlor-2-nitro-benzol (1 g) | 6 | 98,5 | 99,7 |
| 3 | 1-Chlor-3-nitro-benzol (2 g) | 6 | 97,9 | 98,9 |
| 4 | Azobenzol (2 g) | 6 | 98,3 | 99,4 |
| 5 | Hydrazobenzol (2 g) | 6 | 97,8 | 98,3 |
| 6 | Azoxybenzol (2 g) | 6 | 98,7 | 99,5 |
| 7 | Kaliumnitrit (5 g) | 6 | 98,8 | 99,2 |

### Beispiel 8

### Herstellung von 4,4'-Bis(naphth-1-yloxy)benzophenon (Vergleich)

Die Reaktion wurde analog Beispiel 1 durchgeführt. Zur Anwendung kamen: 120 ml NMP, 31,38 g (0,125 mol) 4,4'-Dichlorbenzophenon, 37,0 g (0,257 mol) Naphth-1-ol und 35,5 g (0,257 mol) Kaliumcarbonat. Nach 10 Stunden Reaktionzeit wurde kein weiterer Reaktionsfortgang beobachtet, und es wurde aufgearbeitet. Die HPLC-Analyse einer Probe ergab 69,4 % 4,4'-Bis(naphth-1-yloxy)benzophenon, 27,2 % 4-(Naphth-1-yloxy)-4'-chlorbenzophenon und 3,4 % nicht identifizierte Nebenprodukte.

### Beispiele 9 bis 14 (erfindungsgemäß)

Man verfuhr analog Beispiel 8, führte jedoch die Umsetzung in Gegenwart der in der folgenden Tabelle 2 aufgeführten Katalysatoren durch.

**Tabelle 2**

| Beispiel Nr. | Katalysator | Reaktionszeit [h] | Ausbeute [% ] | Reinheit (HPLC) [%] |
|---|---|---|---|---|
| 9 | 1-Chlor-2-nitrobenzol (1 g) | 8 | 97,3 | 98,8 |
| 10 | 1-Chlor-3-nitrobenzol (2 g) | 8 | 96,6 | 98,3 |
| 11 | Azobenzol (2 g) | 8 | 96,3 | 97,9 |
| 12 | Hydrazobenzol (2 g) | 8 | 96,6 | 97,9 |
| 13 | Azoxybenzol (2 g) | 8 | 98,1 | 98,9 |
| 14 | Kaliumnitrit (5 g) | 8 | 98,1 | 98,9 |

### Beispiel 15

### Herstellung von 4,4'-Bis(biphenyloxy)benzophenon (Vergleich)

Man verfuhr analog Beispiel 1. Zur Anwendung kamen: 250 ml NMP, 31,4 g (0,125 mol) 4,4'-Dichlorbenzophenon, 42,5 g 4-Hydroxybiphenyl und 26,0 g (0,26 mol) Kaliumcarbonat. Nach 10 Stunden Reaktionszeit war kein weiterer Reaktionsfortgang zu erkennen, und es wurde aufgearbeitet. Die HPLC-Analyse einer Probe zeigte 71,4 % 4,4'-Bis(biphenyloxy)benzophenon, 25,8 % 4-Biphenyloxy-4'-chlorbenzophenon und 2,8 % nicht identifizierter Nebenprodukte.

### Beispiele 16 bis 21 (erfindungsgemäß)

Man verfuhr analog Beispiel 15, führte jedoch die Umsetzung in Gegenwart der in der folgenden Tabelle 3 aufgeführten Katalysatoren durch.

**Tabelle 3**

| Beispiel Nr. | Katalysator | Reaktionszeit [h] | Ausbeute [%] | Reinheit (HPLC) [%] |
|---|---|---|---|---|
| 16 | 1-Chlor-2-nitrobenzol (1 g) | 8 | 98,7 | 98,9 |
| 17 | 1-Chlor-3-nitro-benzol (2 g) | 8 | 98,2 | 98,1 |
| 18 | Azobenzol (2 g) | 8 | 97,3 | 97,9 |
| 19 | Hydrazobenzol (2 g) | 8 | 96,9 | 97,4 |
| 20 | Azoxybenzol (2 g) | 8 | 98,7 | 98,8 |
| 21 | Kaliumnitrit (5 g) | 8 | 98,5 | 98,9 |

### Beispiel 22

### Herstellung von 4,4'-Bis(4-phenoxyphenylsulfonyl)biphenyl (Vergleich)

Man verfuhr analog Beispiel 1. Zur Anwendung kamen: 62,9 g (0,125 mol) 4,4'-Bis(4-chlorphenylsulfonyl)biphenyl, 23,5 g (0,25 mol) Phenol, 36,0 g (0,26 mol) Kaliumcarbonat und 250 ml NMP. Nach 8 Stunden Reaktionszeit war kein weiterer Reaktionsfortgang zu erkennen, und es wurde aufgearbeitet. Die HPLC-Analyse einer Probe ergab 70,3 % 4,4'-Bis(4-phenoxyphenylsulfonyl)biphenyl, 27,8 % 4-Phenoxyphenylsulfonyl-4'-chlorphenylsulfonylbiphenyl und 1,9 % nicht identifizierte Nebenprodukte.

### Beispiele 23 bis 28 (erfindungsgemäß)

Man verfuhr analog Beispiel 22, führte jedoch die Umsetzung in Gegenwart der in der folgenden Tabelle 4 aufgeführten Katalysatoren durch.

**Tabelle 4**

| Beispiel Nr. | Katalysator | Reaktionszeit [h] | Ausbeute [%] | Reinheit (HPLC) [%] |
|---|---|---|---|---|
| 23 | 1-Chlor-2-nitrobenzol (1 g) | 5 | 97,9 | 97,7 |
| 24 | 1-Chlor-3-nitrobenzol (2 g) | 5 | 97,5 | 97,5 |
| 25 | Azobenzol (2 g) | 5 | 97,0 | 96,9 |
| 26 | Hydrazobenzol (2 g) | 5 | 98,0 | 97,2 |
| 27 | Azoxybenzol (2 g) | 5 | 98,7 | 98,5 |
| 28 | Kaliumnitrit (5 g) | 5 | 98,3 | 98,4 |

## Patentansprüche

1. Verfahren zur Herstellung von Arylethern der Formel I 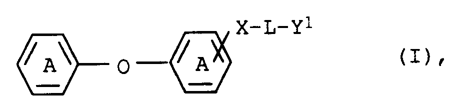 in der
der Ring A gegebenenfalls substituiert ist und benzoanelliert sein kann,
X Carbonyl oder Sulfonyl,
L eine chemische Bindung oder ein Brückenglied und
Y¹
a) für den Fall, daß L für ein Brückenglied steht, Halogen oder den Rest der Formel 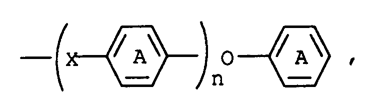 worin n die Bedeutung von 0 oder 1 und X und der Ring A jeweils die obengenannte Bedeutung besitzen,
b) für den Fall, daß L für eine chemische Bindung und X für Carbonyl stehen, Wasserstoff, C₁-C₄-Alkyl, C₁-C₄-Alkoxy oder den Rest der Formel 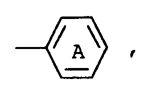 worin der Ring A die obengenannte Bedeutung besitzt, oder
c) für den Fall, daß L für eine chemische Bindung und X für Sulfonyl stehen, den Rest der Formel 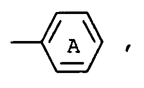 worin der Ring A die obengenannte Bedeutung besitzt,
bedeuten, durch Umsetzung von Hydroxyaromaten der Formel II 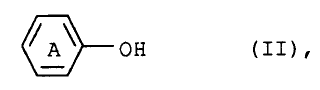 in der der Ring A die obengenannte Bedeutung besitzt, mit Halogenverbindungen der Formel III 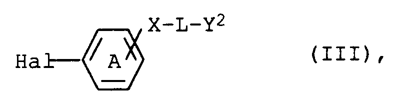 in der
Hal Chlor oder Brom und
Y² einen Rest der Formel 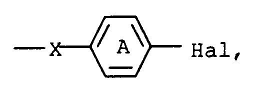
worin X, Hal und der Ring A jeweils die obengenannte Bedeutung besitzen, oder den obengenannten Rest Y¹ bedeuten und X, Hal und der Ring A jeweils die obengenannte Bedeutung besitzen, in einem dipolar aprotischen Lösungsmittel in Gegenwart einer Base, dadurch gekennzeichnet, daß man die Umsetzung bei einer Temperatur von 60 bis 240°C zusätzlich in Gegenwart einer katalytischen Menge einer Verbindung vornimmt, die ausgewählt ist aus der Klasse, bestehend aus Alkalinitrit und Verbindungen der Formel IV 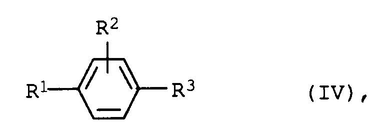 in der
R¹ und R² gleich oder verschieden sind und unabhängig voneinander jeweils Wasserstoff, C₁-C₁₂-Alkyl, das gegebenenfalls durch Phenyl substituiert ist, C₁-C₁₂-Alkoxy, Halogen oder Nitro und
R³ Nitro, Nitroso oder einen Rest der Formel 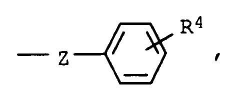 worin R⁴ für Wasserstoff, C₁-C₄-Alkyl oder Halogen und Z für einen Rest der Formel -N=N, 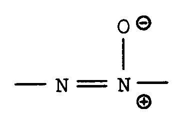 oder -NH-NH- stehen, bedeuten, mit der Maßgabe, daß wenn R³ für Nitro steht, mindestens einer der beiden Reste R¹ und R² die Bedeutung von Halogen besitzt.

2. Verfahren nach Anspruch 1, dadurch gekennzeichnet, daß der Ring A unsubstituiert, durch Phenyl substituiert oder benzoanelliert ist.

3. Verfahren nach Anspruch 1, dadurch gekennzeichnet, daß L ein Brückenglied bedeutet.

4. Verfahren nach Anspruch 1, dadurch gekennzeichnet, daß Hal Chlor bedeutet.

5. Verfahren nach Anspruch 1, dadurch gekennzeichnet, daß man Arylether der Formel Ia 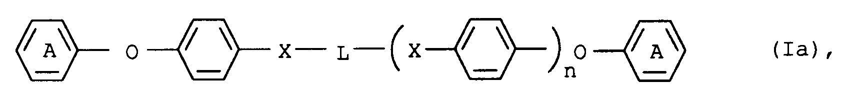 in der
der Ring A unsubstituiert, durch Phenyl substituiert oder benzoanelliert ist,
X Carbonyl oder Sulfonyl,
L Phenylen oder Biphenylen und
n 0 oder 1 bedeuten, durch Umsetzung von Hydroxyaromaten der Formel II 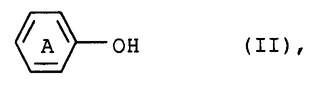 in der der Ring A die obengenannte Bedeutung besitzt, mit Halogenverbindungen der Formel IIIa 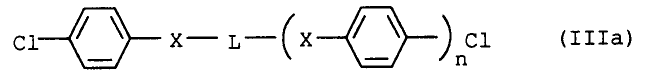 in der X, L und n jeweils die obengenannte Bedeutung besitzen, herstellt.

6. Verfahren nach Anspruch 1, dadurch gekennzeichnet, daß man die Umsetzung in Gegenwart einer Verbindung der Formel IV durchführt.
